# EUROPÄISCHE PATENTSCHRIFT

(11) **EP 0 587 050 B1**
(45) Veröffentlichungstag und Bekanntmachung des Hinweises auf die Patenterteilung: **13.03.1996**
(21) Anmeldenummer: 93114062.8
(22) Anmeldetag: 02.09.1993
(51) Int. Cl.: C07C 253/30, C07C 67/347, C07C 255/50, B01J 31/18

(54) **Verfahren zur Herstellung von aromatisch substituierten Olefinen aus Chloraromaten**
Process for the preparation of an aromatic group substituted olefins from chloroaromatics
Procédé de préparation d'oléfines substituées par un groupe aromatique à partir de composés chloroaromatiques

(30) Priorität: 09.09.1992 DE 4230107
(43) Veröffentlichungstag der Anmeldung: 16.03.1994
(73) Patentinhaber: HOECHST AKTIENGESELLSCHAFT, D-65929 Frankfurt am Main (DE)
(72) Erfinder: Beller, Matthias, Dr., D-65527 Niedernhausen (DE); Fischer, Hartmut, D-65719 Hofheim (DE); Strutz, Heinz, Dr., D-61250 Usingen (DE)

(56) Entgegenhaltungen:
- EP-A- 0 103 544
- EP-A- 0 406 848
- JOURNAL OF ORGANOMETALLIC CHEMISTRY, Band 270, 1984, Lausanne A. SPENCER "Homogenous palladuim-catalysed arylation of activated alkenes with aryl chlorides"

## Beschreibung

Die vorliegende Erfindung betrifft ein Verfahren zur Herstellung von aromatisch substituierten Olefinen durch katalytische Arylierung von Olefinen mit Chloraromaten.

Aromatisch substituierte Olefine sind wichtige Monomere für die Synthese von Polymeren und stellen bedeutende Zwischenprodukte zur Herstellung von Pharmazeutika und Agrochemikalien dar. So finden Stilbene als optische Aufheller, Styrole zur Herstellung von Polymeren und Zimtsäureester als UV-Absorber in Kosmetika technische Verwendung.

Die Herstellung von aromatischen Olefinen durch palladiumkatalysierte Olefinierung eines Jod- oder Bromaromaten in Gegenwart einer Base ist als HECK-Reaktion seit langem bekannt. Dagegen gelingt die Olefinierung analoger Chloraromaten nur in schlechten bis mäßigen Ausbeuten oder es werden extrem labile Katalysatorsysteme benutzt, die eine technische Umsetzung schwierig und kompliziert gestalten.

In J. Organomet. Chem. 270 (1984) S.115 ist die Vinylierung von Chloraromaten in Gegenwart von Palladiumacetat und Triphenylphosphan als Liganden beschrieben. Nach dieser Methode werden jedoch selbst bei den reaktivsten Chloraromaten, also solchen mit elektronenziehenden Substituenten, nur mäßige Ausbeuten bis maximal 51 % erhalten. Für Chlorbenzol als nichtaktiviertem Aromaten ergaben sich sogar nur Ausbeuten von 4 % Eine weitere Schwierigkeit besteht darin, daß der Katalysator desaktiviert wird, was zu maximal 51 Reaktionszyklen führt.

In der EP-A-103 544 wird ein Verfahren beschrieben, bei dem Chloraromaten mit Olefinen in Gegenwart eines Palladiumkatalysators, der Phosphor- oder Arsenliganden enthält, umgesetzt werden. Die Ausbeuten betragen jedoch maximal 61 % für aktivierte Chloraromaten und sind für nichtaktivierte Chloraromaten noch deutlich schlechter.

Gemäß der US-Patentschrift 4 814 489 werden Chloraromaten mit Iodid- und Bromidsalzen in Gegenwart eines Nickelkatalysators zu Arylbromiden bzw. - iodiden umgesetzt, die dann ihrerseits in Gegenwart eines Palladiumkatalysators zu aromatischen Olefinen umgesetzt werden. Bei diesem Verfahren ist es jedoch von Nachteil, daß stöchiometrische Mengen an Iodid- oder Bromidsalzen benötigt werden. Außerdem gestaltet sich bei der Verwendung von zwei Katalysatorsystemen ein Recycling der Katalysatoren besonders schwierig.

Aus der EP-A 406 848 ist die katalytische Formylierung von Chloraromaten in Gegenwart chelatisierender Bisphosphane der Formel R₁R₂P(CH₂)ₙPR₃R₄, mit n = 3 oder 4 bekannt. Nachteile dieses Verfahrens bestehen in der extremen Empfindlichkeit der Liganden und des Katalysators, die ein Katalysatorrecycling schwierig machen und die Desaktivierung des Katalysators durch Palladiumabscheidung begünstigen.

In Bull. Soc. Chem. France, 1973, S.2790 wird eine heterogen katalysierte Arylierung von Styrolen mit Chloraromaten beschrieben. Allerdings sind die Ausbeuten und Umsätze schlecht. Andere Olefine lassen sich nach diesem Verfahren nicht oder nur sehr schwer arylieren.

Aufgabe der vorliegenden Erfindung war es nun, ein Verfahren zur katalytischen Olefinierung von Chloraromaten bereitzustellen, das die vorstehend beschriebenen Nachteile nicht aufweist und das insbesondere aromatische Olefine, wie Styrole, Stilbene und Zimtsäureester in möglichst hoher Ausbeute ohne störende Verunreinigungen und in einfacher Weise liefert und zusätzlich eine einfache technische Umsetzung erlaubt. Insbesondere sollte ein einfaches, stabiles Katalysatorsystem entwickelt werden, das Chloraromaten ähnlich effektiv wie Brom- oder Iodaromaten aktiviert.

Die Lösung dieser Aufgabe gelingt erfindungsgemäß durch ein Verfahren zur Herstellung von aromatisch substituierten Olefinen der Formel (I) 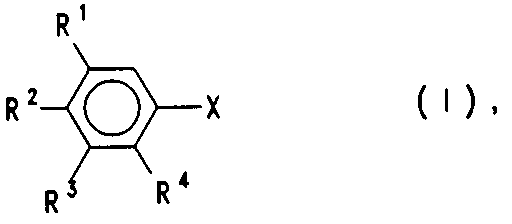
worin X C₂₋₁₂-Alkenyl, C₅₋₆-Cycloalkenyl oder eine Gruppe 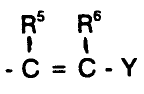
darstellt,
- Y: -CN, -COOH, -COOR⁷, -CON(R⁷)₂, -COR⁷, -OR⁷ oder 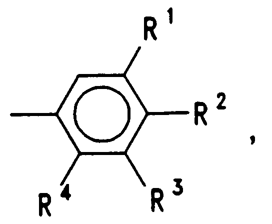
- R⁵: Wasserstoff oder Methyl,
- R⁶: Wasserstoff oder Methyl,
- R⁷: C₁₋₁₂-Alkyl oder Phenyl und
- R²: Wasserstoff, Phenyl, C₁₋₈-Alkyl, C₁₋₅-Alkoxy, -OH, Fluor, Chlor, -NO₂, -CN, -CHO, -CO-C₁₋₄-Alkyl, -CO-Phenyl, -COO-C₁₋₄-Alkyl, -O-CO-C₁₋₄-Alkyl, -NHCO-C₁₋₄-Alkyl, -CF₃, -NH₂, -NH-C₁₋₄-Alkyl oder -N(C₁₋₄-Alkyl)₂ bedeuten,
- R¹ und R³: unabhängig voneinander die gleiche Bedeutung wie R² haben können und
- R⁴: Wasserstoff, Phenyl, C₁₋₈-Alkyl, -OH, Fluor, Chlor, -NO₂, -CN, -CHO oder -O-CO-C₁₋₄-Alkyl darstellt, durch Umsetzung von Chloraromaten der Formel (II) 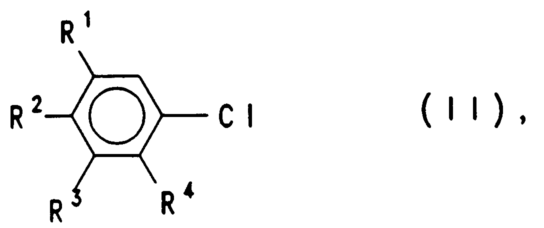
worin R¹, R², R³ und R⁴ die genannte Bedeutung haben, mit Olefinen der Formel (III)

HX (III),

worin X die genannte Bedeutung hat, in Gegenwart einer Base und katalytischer Mengen eines Palladiumkomplexes, das dadurch gekennzeichnet ist, daß der Palladiumkomplex ein- und zweizähnige Phosphane als Liganden enthält.

Stellt X in Formel (III) Alkenyl mit 3 - 12 C-Atomen oder C₄₋₈-Cycloalkenyl dar, so kann bei der Reaktion eine Verschiebung der Doppelbindung eintreten, und es entstehen im allgemeinen Isomerengemische.

Bevorzugt stellt mindestens einer der Reste R⁵ und R⁶ Wasserstoff dar.

Alkylgruppen R⁷ und R¹ bis R⁴, Alkoxygruppen R¹ bis R³ sowie Alkylgruppen in den Substituenten R¹ bis R⁴ können geradkettig oder verzweigt sein. Als Beispiele für die Gruppen R¹ bis R³ seien genannt: Methyl, Ethyl, n-Propyl, Isopropyl, n-Butyl, sek.-Butyl, n-Pentyl, 2-Pentyl, n-Hexyl, n-Heptyl, n-Octyl, n-Decyl; Methoxy, Ethoxy, n-Propoxy, Isopropoxy, n-Butoxy, n-Pentyloxy; die Carbonsäuremethyl-, -ethyl-, -n-propyl-, -isopropyl-, -n-butyl- und sek.-butylgruppe; die Acetamid-, Propionamid-, Butyramid- und Valeramidgruppe; die N-Methylamino-, N-Ethylamino-, N-n-Propylamino- und N-n-Butylaminogruppe; die N,N-Dimethylamino-, N,N-Diethylamino-, N,N-Di-n-propylamino, N,N-Di-n-butylamino-, N-Methyl-N-ethylamino-, N-Methyl-N-n-propylamino-, N-Ethyl-N-n-butylaminogruppe und die Acetoxy-, Propionyloxy- und Butyryloxygruppe.

Alkylgruppen R⁷ weisen bevorzugt 1 - 10 und insbesondere 1 - 8 C-Atome auf. Besonders bevorzugt bedeutet R⁷ Methyl, Ethyl, n-Butyl oder 2-Ethyl-hexyl.

Alkylgruppen R¹ bis R³ sind bevorzugt geradkettig und weisen 1 - 4, insbesondere 1 oder 2 C-Atome auf. Bevorzugte Alkoxygruppen R¹ bis R³ sind die Methoxy- und Ethoxygruppe.

Stellt Y eine Gruppe 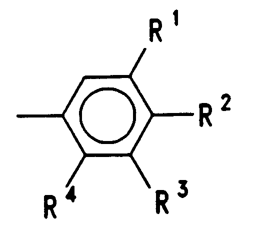
dar, so können deren Substituenten
R¹, R², R³ und R⁴ dieselben wie die entsprechenden Substituenten an der Verbindung der Formel (II) oder davon verschieden sein, wobei symmetrische oder asymmetrische Stilbenderivate entstehen. Stilbenderivate können auch dadurch hergestellt werden, daß man als Verbindung der Formel (III) Ethylen mit gleichen oder verschiedenen Chloraromaten der Formel (II) umsetzt.

Bevorzugt verwendet man Chloraromaten der Formel (II), worin R⁴ Wasserstoff und R¹ bis R³ unabhängig voneinander Wasserstoff, Phenyl, C₁₋₃-Alkyl, insbesondere Methyl, Methoxy, Acetoxy, -NO₂, -CN, -CHO, Cl, -NHCO-C₁₋₂-Alkyl, -COO-C₁₋₂-Alkyl, -CO-C₁₋₂-Alkyl, -CO-Phenyl oder -N(C₁₋₂-Alkyl)₂ bedeuten.

Besonders bevorzugt verwendet man Verbindungen der Formel (II), worin R¹, R³ und R⁴ Wasserstoff oder Methyl und R² Wasserstoff, Phenyl, Methyl, Methoxy, Acetoxy, -CN, -NO₂, -COOC₁₋₂-Alkyl, -CHO, -Cl oder -COCH₃ darstellen.

Als Verbindungen der Formel (III) verwendet man vorzugsweise solche, worin X Alkenyl mit 2 - 8 C-Atomen, Cyclopentenyl und Cyclohexenyl oder eine Gruppe -C(R⁵)=C(R⁶)-Y darstellt, wobei eines von R⁵ und R⁶ Wasserstoff und das andere Methyl oder beide Wasserstoff sind, und Y Phenyl, -CN, -CON(R⁷)₂ oder -COOR⁷ mit R⁷ = Phenyl oder C₁₋₈-Alkyl, insbesondere Methyl oder Ethyl darstellen. Besonders bevorzugt verwendet man als Verbindung der Formel (III) Styrol, Ethylen, Propylen, Acrylnitril, Acrylsäuremethyl, -ethyl-, -butyl- oder -2-ethylhexylester, N,N-Dimethyl- und N,N-Diethylacrylamid. Ganz besonders bevorzugt ist die Herstellung von 4-Methoxy-, 4-Acetyl-, 4-Formyl-, 4-Nitro-, 4-Cyanozimtsäurebutylester und -ethylhexyl, 4,4'-Dinitro-, 4-Cyano-, 4,4'-Dicyanostilben und 4-Cyanostyrol.

Bei der Umsetzung mit Ethylen können Styrol- und/oder Stilbenderivate entstehen. Die enindungsgemäße Umsetzung kann bei Normaldruck oder unter Druck, z. B. einem Druck von 1 bis 60 bar, durchgeführt werden. Die Reaktion läßt sich hauptsächlich durch Variation des angewendeten Druckes steuern. Stilbene werden vornehmlich bei Normaldruck gebildet, während bei höherem Druck, insbesondere einem Druck zwischen 5 und 40 bar, zur Hauptsache Styrolderivate entstehen.

Die Reaktionstemperaturen liegen vorzugsweise zwischen 120 und 200 °C, insbesondere zwischen 140 und 180 °C.

Bei dem Palladiumkomplex, der bei dem erfindungsgemäßen Verfahren als Katalysator eingesetzt wird, handelt es sich um Palladium-Phosphan-Komplexe, bei denen das Palladium durch eine Kombination aus einzähnigen und chelatisierenden zweizähnigen Phosphanliganden komplexiert ist. Diese spezielle Kombination von Liganden führt zu einem stabileren und zugleich kinetisch aktiveren Katalysatorkomplex, so daß ein sehr hoher Umsatz der Chloraromaten erreicht wird. Als Katalysatoren wirken Palladium-Phosphan-Komplexe, die aus üblichen Palladium(II)-Salzen bzw. -komplexen und/oder Palladium(0)-Komplexen und einer Kombination von einzähnigen und chelatisierenden zweizähnigen Phosphanen gebildet werden, denen gegebenenfalls zur Steigerung der Selektivität der Olefinierung eine Carbonsäure, wie Ameisensäure oder Essigsäure, zugesetzt werden kann. Als Palladiumsalze bzw. -komplexe kommen beispielsweise Palladiumdichlorid, Palladiumacetat, Palladiumtrifluoracetat, Palladiumdibromid, Palladiumdinitrat, Palladiumdichloriddibenzonitrilkomplex, Bis-[dibenzylidenaceton]palladium(0) und Tetrakis-(triphenylphosphan)palladium(0) in Frage.

In der Reaktionsmischung bildet sich der Katalysatorkomplex spontan aus den Komponenten. In einer bevorzugten Ausführungsform des erfindungsgemäßen Verfahrens wird der Palladium(II)-phosphan-Komplex jedoch separat in Lösung hergestellt und dann zur Reaktionsmischung gegeben, wo unter den Reaktionsbedingungen in situ die Reduktion zum aktiven Palladium(0)-Komplex erfolgt. Der Pd(0)-Phosphankomplex kann zwar ebenfalls separat hergestellt werden, jedoch kompliziert dieser Schritt das Verfahren.

Die Menge der pro Mol Pd-Atome eingesetzten Phosphane liegt im allgemeinen bei wenigstens 0,5 Mol Bisphosphan und 0,5 Mol Monophosphan, was einem P:Pd-Verhältnis von 1,5:1 entspricht. Eine Verminderung des Bisphosphananteils ist prinzipiell möglich, führt aber zu thermisch labileren Katalysatorsystemen, die leicht metallisches Palladium abscheiden. Ebenso ist es nicht zweckmäßig, den Anteil des Bisphosphans über 2 Mol pro Mol Palladiumatome zu steigern. Ein erhöhter Bisphosphananteil verbessert zwar die thermische Stabilität des Katalysatorkomplexes, führt gleichzeitig aber auch zu dessen kinetischer Desaktivierung. Das Monophosphan kann zweckmäßig bis zu einem Verhältnis von 4 Mol pro Mol Palladiumatome eingesetzt werden. Somit beträgt also zweckmäßig das Molverhältnis von Phosphor zu Palladium zwischen 1,5:1 und 8:1. Bevorzugt liegt dieses Verhältnis zwischen 1,6:1 und 6:1, insbesondere zwischen 2:1 und 5:1. Als günstigste Kombination hat sich der Einsatz von 1 Mol Bisphosphan und 2 Mol Monophosphan pro Palladiumatom erwiesen, was einem Verhältnis von Gesamtphosphor zu Palladium von 4 entspricht. Das Molverhältnis von Bisphosphan zu Monophosphan liegt zweckmäßig zwischen 1:1 und 1:4, bevorzugt zwischen 1:1,5 und 1:3. Am günstigsten ist ein Verhältnis von 1:2.

Bei den chelatisierenden zweizähnigen Bisphosphanen handelt es sich im allgemeinen um Bis-(di-C₁₋₆-alkyl-phosphino)- und Bis-(diphenylphosphino)alkane mit Alkanbrücken mit 1 bis 5 C-Atomen oder um Bis-(di-C₁₋₆-alkylphosphino)- und Bis-(diphenylphosphino)aryle, wobei das Aryl 1,2-Phenyl oder 2,3-Naphthyl ist. Dabei können die endständigen Phenylgruppen jeweils mit C₁₋₄-Alkyl, C₁₋₃-Alkoxy oder SO₃Na substituiert sein. Beispielsweise kommen die folgenden Verbindungen in Frage:
Bis-(diisopropylphosphino)methan, -ethan, -propan, -butan; Bis-(di-n-propylphosphino)-butan, Bis-(di-n-butylphosphino)ethan, Bis-(dicyclohexylphosphino)ethan, -propan, -butan; Bis-(diphenylphosphino)methan, -ethan, -propan, -butan; Bis-[di(methoxyphenyl)phosphino]methan, -ethan, - propan, -butan; Bis-(diphenylphosphino)benzol, Bis-(diisopropylphosphino)benzol. Bevorzugt sind Bis-(diphenylphosphino)ethan, Bis-(diphenylphosphino)propan und Bis-(diphenylphosphino)butan.

Als einzähnige Monophosphane kommen insbesondere Triarylphosphane, Dialkylarylphosphane, Diarylalkylphosphane und Trialkylphosphane in Frage, wobei die Alkylgruppen 1 bis 12 C-Atome enthalten und die Arylgruppen Phenyl- oder Naphthylgruppen sind, die jeweils mit C₁₋₄-Alkyl, C₁₋₃-Alkoxy oder SO₃Na substituiert sein können.

Als Beispiele seien genannt:
Triphenylphosphan, Tricyclohexylphosphan, Triisopropylphosphan, Tri-n-butylphosphan, Tri(methoxyphenyl)phosphan, Diisopropylphenylphosphan, Diphenylisopropylphosphan, Triisobutylphosphan, Methyldiphenylphosphan, Tri-o- und p-tolylphosphan, Triethylphosphan, tert. Butyldiphenylphosphan, und Tri(sulfonatophenyl)phosphan.

Besonders bevorzugt sind Triphenylphosphan, Tricyclohexylphosphan und Tri-o-tolyl-phosphan.

Der Katalysatorkomplex, berechnet als einkerniger Palladium-Komplex, wird zweckmäßig in einer Menge von 0,001 bis 10 Mol-%, bevorzugt von 0,1 bis 1 Mol-%, bezogen auf den Chloraromaten, eingesetzt.

Als Basen können z.B. die in der EP-A-103 544 beschriebenen Verbindungen, vor allem Amine, wie Triethylamin, Tripropylamin, Tributylamin, N-Methylmorpholin und Benzyldimethylamin und insbesondere Salze von Carbonsäure, wie die Alkalisalze von Carbonsäuren mit 2 bis 4 C-Atomen eingesetzt werden. Besonders bevorzugt wird Natriumacetat eingesetzt.

Die Ausgangsprodukte der Formel (II) und (III) sind bekannt oder können nach üblichen Methoden hergestellt werden.

Sind die Verbindungen der Formel (II) und (III) flüssig, so kann die Umsetzung ohne Zusatz eines Lösungsmittels durchgeführt werden. Bevorzugt wird die Reaktion jedoch in einem gegenüber den Reaktionspartnern inerten organischen Lösungsmittel durchgeführt. Geeignete inerte organische Lösungsmittel sind je nach Reaktionskomponenten z.B. gegebenenfalls chlorierte aliphatische, cycloaliphatische oder aromatische Kohlenwasserstoffe, wie n-Pentan, n-Heptan, n-Octan, Cyclopentan, Cyclohexan, Benzol, Toluol, Xylole und Chlorbenzol; aromatische, aliphatische und cyclische Ether, wie Anisol, Diethylether, Diisopropylether, Tetrahydrofuran und Dioxan; N-substtuierte Morpholine, wie N-Methyl- und N-Formylmorpholin; Nitrile, besonders Benzonitril und Alkylnitrile mit 2 bis 5 C-Atomen, wie Acetonitril, Propionitril und Butyronitril; 3-Methoxypropionitril und 3-Ethoxypropionitril, Dialkylsulfoxide, wie Dimethyl- und Diethylsulfoxid; N,N-Dialkylamide von aliphatischen Monocarbonsäuren mit 1 bis 3 C-Atomen im Säureteil, wie N,N-Dimethylformamid und N,N-Dimethylacetamid; Alkohole mit bis zu 8 C-Atomen, wie Ethanol, n-Propanol und tert.-Butanol; aliphatische und cyclische Ketone, wie Aceton, Diethylketon, Methylisopropylketon, Cyclopentanon, Cyclohexanon, 1,3-Dimethyl-2-imidazolidinon und 1,3-Dimethyl-3,4,5,6-tetrahydro-2-(1H)-pyrimidinon; Tetramethylharnstoff; Ester, wie Ester der Kohlensäure, z.B. Diethylcarbonat; Nitromethan; Alkyl- oder Alkoxyalkylester von aliphatischen Monocarbonsäuren mit insgesamt 2 bis 8 C-Atomen, wie Essigsäuremethyl-, - ethyl-, -n-butyl- und -isobutylester, Buttersäureethyl- und -n-butylester sowie 1-Acetoxy-2-ethoxyethan. Bevorzugte Lösungsmittel sind N,N-Dialkylamide, besonders Dimethylacetamid, Dimethylformamid, N-Methylpyrrolidon und Ethylenglykol-, Di-, Tri- und Tetraethylenglykoldimethylether.

Die durch die Erfindung erreichten Vorteile sind im wesentlichen darin zu sehen, daß eine einfache allgemeine Methode gefunden wurde, um Chloraromaten in hohen Ausbeuten mit Olefinen umzusetzen und so aromatische Olefine herzustellen.

Ein weiterer Vorteil des erfindungsgemäßen Verfahrens besteht darin, daß das Katalysatorsystem unter den Reaktionsbedingungen aktiv bleibt, es zu keiner Palladiumabscheidung kommt und daher ein Recycling möglich ist.

### Beispiele

Die nachfolgend beschriebenen Versuche wurden unter Argon als Schutzgasatmosphäre durchgeführt. Bei hochsiedenden Ausgangsstoffen sind übliche Rundkolben mit Argonüberlagerung und einem Systemansatz zum Einspritzen der Katalysatorlösung ausreichend. Wenn der Siedepunkt von Ausgangsstoff oder Lösungsmittel deutlich unter 140°C liegt, müssen druckfeste Reaktoren, wie Glasautoklaven oder Edelstahlautoklaven eingesetzt werden.
1. In 50 ml Dimethylacetamid (DMAc) wurden 13,8 g (100 mMol) 4-Chlorbenzonitril und 19,2 g (150 mMol) Acrylsäurebutylester gelöst. Zu dieser Lösung wurden 9,0 g (110 mMol) trockenes und gemahlenes Natriumacetat gegeben. Separat davon wurde eine Lösung der Katalysatorvorstufe aus 112 mg (0,5 mMol) Palladiumacetat, 200 mg (0,5 mMol) 1,2-Bis-(diphenylphosphino)ethan (DIPHOS) und 262 mg (1 mMol) Triphenylphosphan in 10 ml Dimethylacetamid bereitet. Diese gelbe Lösung wurde dann zum Ansatz gegeben, der anschließend für 15 h bei 140°C gerührt wurde. Die Reaktion kann durch GC-Analytik mit internem Standard (N-Methylpyrrolidon oder Ditolylether) gut verfolgt werden. Nach der oben angegebenen Reaktionszeit wurde 100 % Umsatz erreicht. Die Ausbeute an isoliertem p-Cyanozimtsäurebutylester betrug 87 %.
2. Das Beispiel 1 wurde wiederholt, jedoch wurde die Katalysatorvorstufe aus 112 mg (0,5 mMol) Palladiumacetat, 200 mg (0,5 mg) DIPHOS, 280 mg (1 mMol) Tricyclohexylphosphan und 3 g (50 mMol) Essigsäure gebildet. Nach 15stündiger Reaktion bei 140°C wurden 97 % Umsatz und 85 % Ausbeute erreicht.
3. In einen 200 ml-Autoklaven wurden 50 ml DMAc, 13,8 g (100 mMol) 4-Chlorbenzonitril und 9 g (110 mMol) Natriumacetat eingebracht. Nach Spülen mit Argon wurde eine Lösung aus 112 mg (0,5 mMol) Palladiumacetat, 200 mg (0,5 mMol) DIPHOS und 262 mg (1 mMol) Triphenylphosphan in 10 ml DMAc einspritzt. Es wurden 2 bar Ethylen aufgedrückt und 24 h bei 145°C gerührt. Die GC-Analyse belegte 100 % Umsatz. Durch präparative Aufarbeitung (Fällung mit Wasser, Umkristallisation aus DMAc) wurden 62 % 4,4'-Dicyanostilben isoliert, Fp. = 287 - 288°C.
4. Das Beispiel 3 wurde wiederholt, jedoch wurde die Reaktion unter einem Ethylendruck von 50 bar durchgeführt. Die Reaktionslösung enthielt 12 % 4,4'-Dicyanostilben und 78 % 4-Cyanostyrol.
5. 13,8 g (100 mMol) 4-Chlorbenzonitril, 20,8 g (200 mMol) Styrol und 8,2 g (100 mMol) Natriumacetat wurden in 60 ml Tetraethylenglykoldimethylether vorgelegt. Der Katalysator wurde aus 224 mg (1 mMol) Palladiumacetat, 400 mg (1 mMol) DIPHOS und 524 mg (2 mMol) Triphenylphosphan gebildet. Nach 18stündiger Reaktion bei 145°C wurde ein Umsatz von 85 % und eine Ausbeute an 4-Cyanostilben von 70 % erreicht.
6. Das Beispiel 5 wurde wiederholt, jedoch wurden anstelle des Triphenylphosphans 560 mg (2 mMol) Tricyclohexylphosphan zur Katalysatorbildung eingesetzt. Die Reaktion führte zu 100 % Umsatz und einer Ausbeute von 85 % an 4-Cyanostilben.
7. Das Beispiel 1 wurde mit 15,8 g (100 mMol) 4-Nitrochlorbenzol als Ausgangsstoff wiederholt. Es wurden 100 % Umsatz erreicht. Nach Aufarbeitung wurden 85 % 4-Nitrozimtsäurebutylester isoliert.
8. In einer Rührapparatur mit Gaseinleitungsfritte wurden 70 ml DMAc, 15,8 g (100 mMol) 4-Nitrochlorbenzol und 9,0 g (110 mMol) Natriumacetat vorgelegt. Die Katalysator-Vorstufe wurde aus 701 mg (1 mMol) Dichlor-bis(triphenylphosphan)palladium und 400 mg (1 mMol) DIPHOS in 20 ml DMAc gebildet. Um den Palladiumkomplex zu lösen, mußte auf 80°C erwärmt werden. Die Katalysator-Lösung wurde zum Ansatz gegeben, und unter Durchleiten von Ethylen wurde die Temperatur auf 140°C gesteigert. Nach 16 h wurde ein Umsatz von 95 % erreicht. Nach Aufarbeitung und Umkristallisation ergaben sich 65 % 4,4'-Dinitrostilben, Fp. = 313°C.
9. 28,2 g (200 mMol) 4-Chlorbenzaldehyd, 38,4 g (300 mMol) Acrylsäurebutylester, 24,8 g (300 mMol) Natriumacetat und 100 ml DMAc wurden vorgelegt. Die Lösung der Katalysatorvorstufe wurde aus 224 mg (1 mMol) Palladiumacetat, 400 mg DIPHOS, 524 mg (2 mMol) Triphenylphosphan und 3 g (50 mMol) Essigsäure in 20 ml DMAc gebildet und zum Ansatz gegeben. Die Reaktion wurde bei 140°C für 24 h durchgeführt. Es wurde 100 % Umsatz bei einer Ausbeute von 92 % an 4-Formylzimtsäurebutylester erreicht.
10. 15,5 g (100 mMol) 4-Chloracetophenon, 25,6 g (200 mMol) Acrylsäurebutylester, 9,0 g (110 mMol) Natriumacetat und 50 ml DMAc wurden vorgelegt. Der Palladiumkatalysator wurde wie in Beispiel 8 hergestellt. Die Reaktion wurde bei 160°C durchgeführt. Schon nach 6 h wurde 100 % Umsatz erreicht, die Ausbeute an 4-Acetylzimtsaurebutylester betrug 92 %.
11. 14,2 g (100 mMol) 4-Chloranisol, 25,6 g (200 mMol) Acrylsäurebutylester, 9,0 g (110 mMol) Natriumacetat und 60 ml N-Methylpyrrolidon wurden vorgelegt. In 20 ml des gleichen Lösungsmittels wurde die Katalysatorlösung aus 224 mg (1 mMol) Palladiumacetat, 400 mg (1 mMol) DIPHOS und 524 mg (2 mMol) Triphenylphosphan gebildet. Die Reaktion wurde bei 180°C durchgeführt. Nach 6 h wurde quantitativer Umsatz und eine Ausbeute von 87 % an 4-Methoxyzimtsäurebutylester erreicht.

## Patentansprüche

1. Verfahren zur Herstellung von aromatisch substituierten Olefinen der Formel (I) 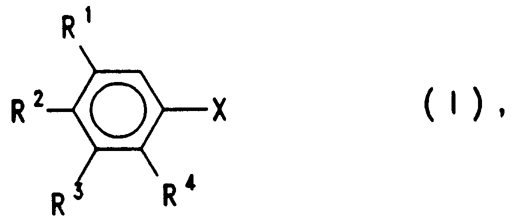 worin X C₂₋₁₂-Alkenyl, C₅₋₆-Cycloalkenyl oder eine Gruppe 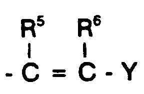 darstellt,
Y -CN, -COOH, -COOR⁷, -CON(R⁷)₂, -COR⁷, -OR⁷ oder 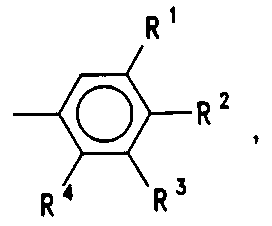
R⁵ Wasserstoff oder Methyl,
R⁶ Wasserstoff oder Methyl,
R⁷ C₁₋₂-Alkyl oder Phenyl und
R² Wasserstoff, Phenyl, C₁₋₈-Alkyl, C₁₋₅-Alkoxy, -OH, Fluor, Chlor, -NO₂, -CN, -CHO, -CO-C₁₋₄-Alkyl, -CO-Phenyl, -COO-C₁₋₄-Alkyl, -O-CO-C₁₋₄-Alkyl, -NHCO-C₁₋₄-Alkyl, -CF₃, -NH₂, -NH-C₁₋₄-Alkyl oder -N(C₁₋₄-Alkyl)₂ bedeuten,
R¹ und R³ unabhängig voneinander die gleiche Bedeutung wie R² haben können und
R⁴ Wasserstoff, Phenyl, C₁₋₈-Alkyl, -OH, Fluor, Chlor, -NO₂, -CN, -CHO oder -O-CO-C₁₋₄-Alkyl darstellt, durch Umsetzung von Chloraromaten der Formel (II) 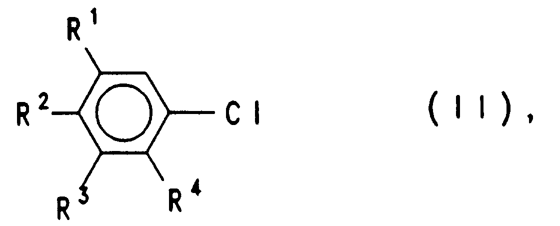
worin R¹, R², R³ und R⁴ die genannte Bedeutung haben, mit Olefinen der Formel (III)
HX (III),
worin X die genannte Bedeutung hat, in Gegenwart einer Base und katalytischer Mengen eines Palladiumkomplexes, dadurch gekennzeichnet, daß der Palladiumkomplex ein- und zweizähnige Phosphane als Liganden enthält.

2. Verfahren nach Anspruch 1, dadurch gekennzeichnet, daß man ein Olefin der Formel (III) verwendet, worin X -C(R⁵)=C(R⁶)-Y darstellt und mindestens einer der Reste R⁵ und R⁶ Wasserstoff ist.

3. Verfahren nach Anspruch 1 oder 2, dadurch gekennzeichnet, daß man eine Verbindung der Formel (II) verwendet, worin R⁴ Wasserstoff und R¹ bis R³ unabhängig voneinander Wasserstoff, Phenyl, Methyl, Methoxy, Acetoxy, -NO₂, -CN, -CHO, Cl, -NHCO-C₁₋₂-Alkyl, -COO-C₁₋₂-Alkyl, -CO-C₁₋₂-Alkyl, -CO-Phenyl oder -N(C₁₋₂-Alkyl)₂ bedeuten.

4. Verfahren nach Anspruch 1 oder 2, dadurch gekennzeichnet, daß man eine Verbindung der Formel (II) verwendet, worin R¹, R³ und R⁴ Wasserstoff oder Methyl und R² Wasserstoff, Phenyl, Methyl, Methoxy, Acetoxy, -CN, -NO₂, -COOC₁₋₂-Alkyl, -CHO, -Cl oder -COCH₃ darstellen.

5. Verfahren nach einem oder mehreren der Ansprüche 1 bis 4, dadurch gekennzeichnet, daß man als Verbindung der Formel (III) Styrol, Ethylen, Propylen, Acrylnitril, Acrylsäuremethyl, -ethyl-, -butyl- oder -2-ethylhexylester, N,N-Dimethyl- oder oder N,N-Diethylacrylamid verwendet.

6. Verfahren nach einem oder mehreren der Ansprüche 1 bis 5, dadurch gekennzeichnet, daß man 4-Methoxy-, 4-Acetyl-, 4-Formyl-, 4-Nitro- oder 4-Cyanozimtsäurebutylester und -ethylhexylester, 4,4'-Dinitro-, 4-Cyano- oder 4,4'-Dicyanostilben oder 4-Cyanostyrol herstellt.

7. Verfahren nach einem oder mehreren der Ansprüche 1 bis 6, dadurch gekennzeichnet, daß man die Reaktion bei einer Temperatur zwischen 120 und 200°C, vorzugsweise zwischen 140 und 180°C, durchführt.

8. Verfahren nach einem oder mehreren der Ansprüche 1 bis 7, dadurch gekennzeichnet, daß der Palladiumkomplex aus Palladium(II)-Salzen und/oder Palladium(0)-Komplexen und ein- und zweizähnigen Phosphanen in der Reaktionsmischung gebildet wird.

9. Verfahren nach einem oder mehreren der Ansprüche 1 bis 7, dadurch gekennzeichnet, daß der Palladiumkomplex aus Palladium(II)-Salzen und/oder Palladium(0)-Komplexen und ein- und zweizähnigen Phosphanen separat hergestellt wird, und der erhaltene Palladium(II)-Phosphan-Komplex in situ zum entsprechenden Palladium(0)-Phosphan-Komplex reduziert wird.

10. Verfahren nach einem oder mehreren der Ansprüche 1 bis 9, dadurch gekennzeichnet, daß das Molverhältnis von Phosphoratomen zu Palladiumatomen in dem Katalysator zwischen 1,5:1 und 8:1, vorzugsweise zwischen 1,6:1 und 6:1, insbesondere zwischen 2:1 und 5:1, liegt.

11. Verfahren nach einem oder mehreren der Ansprüche 1 bis 10, dadurch gekennzeichnet, daß man einen Katalysator einsetzt, bei dem das Molverhältnis von einzähnigen Phosphanen zu Palladiumatomen zwischen 0,5:1 und 4:1 und von zweizähnigen Bisphosphanen zu Palladiumatomen zwischen 0,5:1 und 2:1 liegt.

12. Verfahren nach einem oder mehreren der Ansprüche 1 bis 11, dadurch gekennzeichnet, daß das Molverhältnis von zweizähnigen Bisphosphanen zu einzähnigen Phosphanen zwischen 1:1 und 1:4, bevorzugt zwischen 1:1,5 und 1:3, insbesondere bei 1:2, liegt.

13. Verfahren nach einem oder mehreren der Ansprüche 1 bis 12, dadurch gekennzeichnet daß man einen Palladiumkomplex einsetzt, der als zweizähnigen Phosphanliganden Bis-(diphenylphosphino)ethan, Bis-(diphenylphosphino)propan oder Bis-(diphenylphosphino)butan enthält.

14. Verfahren nach einem oder mehreren der Ansprüche 1 bis 13, dadurch gekennzeichnet, daß man einen Palladiumkomplex einsetzt, der als einzähnigen Phosphanliganden Triphenylphosphan, Tricyclohexylphosphan oder Tri-o-tolylphosphan enthält.

15. Verfahren nach einem oder mehreren der Ansprüche 1 bis 14, dadurch gekennzeichnet, daß der Palladiumkomplex, berechnet als einkerniger Palladiumkomplex, in einer Menge von 0,001 bis 10 Mol-%, bevorzugt von 0,1 bis 1 Mol-%, bezogen auf den Chloraromaten(II), eingesetzt wird.

16. Verfahren nach einem oder mehreren der Ansprüche 1 bis 15, dadurch gekennzeichnet, daß als Base Natriumacetat eingesetzt wird.

## Claims

1. A process for the preparation of aromatically substituted olefins of the formula (I) 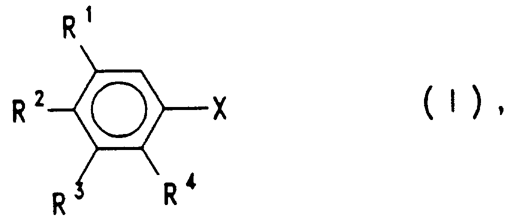 in which X is C₂₋₁₂-alkenyl, C₅₋₆-cycloalkenyl or a group 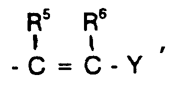 Y is -CN, -COOH, -COOR⁷, -CON(R⁷)₂, -COR⁷, -OR⁷ or 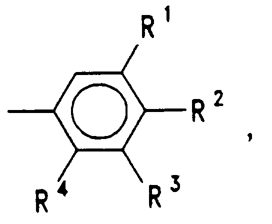
R⁵ is hydrogen or methyl,
R⁶ is hydrogen or methyl,
R⁷ is C₁₋₁₂-alkyl or phenyl and
R² is hydrogen, phenyl, C₁₋₈-alkyl, C₁₋₅-alkoxy, -OH, fluorine, chlorine, -NO₂, -CN, -CHO, -CO-C₁₋₄-alkyl, -CO-phenyl, -COO-C₁₋₄-alkyl, -O-CO-C₁₋₄-alkyl, -NHCO-C₁₋₄-alkyl, -CF₃, -NH₂, -NH-C₁₋₄-alkyl or -N(C₁₋₄-alkyl)₂,
R¹ and R³ independently of one another can have the same meaning as R² and
R⁴ is hydrogen, phenyl, C₁₋₈-alkyl, -OH, fluorine, chlorine, -NO₂, -CN, -CHO or -O-CO-C₁₋₄-alkyl, by reaction of chloroaromatics of the formula (II) 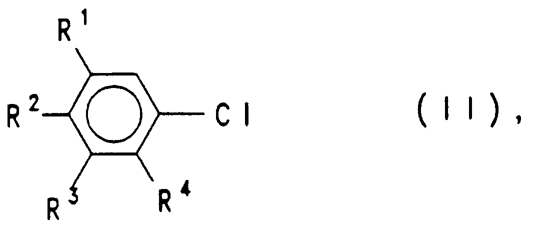
in which R¹, R², R³ and R⁴ have the meaning mentioned, with olefins of the formula (III)
HX (III),
in which X has the meaning mentioned, in the presence of a base and catalytic amounts of a palladium complex, wherein the palladium complex contains mono- and bidentate phosphanes as ligands.

2. The process as claimed in claim 1, wherein an olefin of the formula (III) is used in which X is -C(R⁵)=C(R⁶)-Y and at least one of the radicals R⁵ and R⁶ is hydrogen.

3. The process as claimed in claim 1 or 2, wherein a compound of the formula (II) is used in which R⁴ is hydrogen and R¹ to R³ independently of one another are hydrogen, phenyl, methyl, methoxy, acetoxy, -NO₂, -CN, -CHO, -Cl, -NHCO-C₁₋₂-alkyl, -COO-C₁₋₂-alkyl, -CO-C₁₋₂-alkyl, -CO-phenyl or -N(C₁₋₂-alkyl)₂.

4. The process as claimed in claim 1 or 2, wherein a compound of the formula (II) is used in which R¹, R³ and R⁴ are hydrogen or methyl and R² is hydrogen, phenyl, methyl, methoxy, acetoxy, -CN, -NO₂, -COOC₁₋₂-alkyl, -CHO, -Cl or -COCH₃.

5. The process as claimed in one or more of claims 1 to 4, wherein the compound of the formula (III) used is styrene, ethylene, propylene, acrylonitrile, methyl, ethyl, butyl or 2-ethylhexyl acrylate, N,N-dimethylacrylamide or N,N-diethylacrylamide.

6. The process as claimed in one or more of claims 1 to 5, wherein butyl and ethylhexyl 4-methoxy-, 4-acetyl-, 4-formyl-, 4-nitro- or 4-cyanocinnamate, 4,4'-dinitro-, 4-cyano- or 4,4'-dicyanostilbene or 4-cyanostyrene are prepared.

7. The process as claimed in one or more of claims 1 to 6, wherein the reaction is carried out at a temperature between 120 and 200°C, preferably between 140 and 180°C.

8. The process as claimed in one or more of claims 1 to 7, wherein the palladium complex is formed in the reaction mixture from palladium(II) salts and/or palladium(0) complexes and mono- and bidentate phosphanes.

9. The process as claimed in one or more of claims 1 to 7, wherein the palladium complex is separately prepared from palladium(II) salts and/or palladium(0) complexes and mono- and bidentate phosphanes, and the palladium(II)-phosphane complex obtained is reduced in situ to the corresponding palladium(0)-phosphane complex.

10. The process as claimed in one or more of claims 1 to 9, wherein the molar ratio of phosphorus atoms to palladium atoms in the catalyst is between 1.5:1 and 8:1, preferably between 1.6:1 and 6:1, in particular between 2:1 and 5:1.

11. The process as claimed in one or more of claims 1 to 10, wherein a catalyst is employed in which the molar ratio of monodentate phosphanes to palladium atoms is between 0.5:1 and 4:1 and of bidentate bisphosphanes to palladium atoms is between 0.5:1 and 2:1.

12. The process as claimed in one or more of claims 1 to 11, wherein the molar ratio of bidentate bisphosphanes to monodentate phosphanes is between 1:1 and 1:4, preferably between 1:1.5 and 1:3, in particular 1:2.

13. The process as claimed in one or more of claims 1 to 12, wherein a palladium complex is employed which contains as bidentate phosphane ligands bis(diphenylphosphino)ethane, bis(diphenylphosphino)propane or bis(diphenylphosphino)butane.

14. The process as claimed in one or more of claims 1 to 13, wherein a palladium complex is employed which contains as monodentate phosphane ligands triphenylphosphane, tricyclohexylphosphane or tri-o-tolylphosphane.

15. The process as claimed in one or more of claims 1 to 14, wherein the palladium complex, calculated as mononuclear palladium complex, is employed in an amount of from 0.001 to 10 mol %, preferably from 0.1 to 1 mol %, relative to the chloroaromatic(II).

16. The process as claimed in one or more of claims 1 to 15, wherein sodium acetate is employed as the base.

## Revendications

1. Procédé pour la préparation d'oléfines à substitution aromatique de formule (I) : 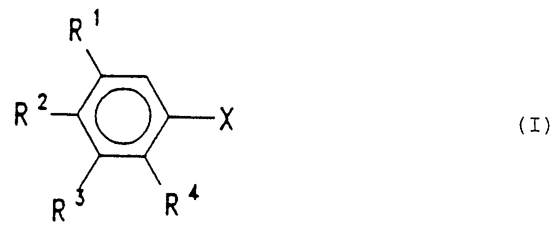 où X représente alcényle en C₂-C₁₂, cycloalcényle en C₅-C₆ ou un groupe 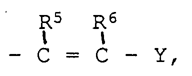
Y représente -CN, -COOH, -COOR⁷, -CON(R⁷)₂, -COR⁷, -OR⁷ ou 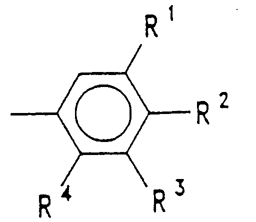
R⁵ représente l'hydrogène ou méthyle,
R⁶ représente l'hydrogène ou méthyle,
R⁷ représente alkyle en C₁₋₁₂ ou phényle et
R² représente l'hydrogène, phényle, alkyle en C₁₋₈, alcoxy en C₁₋₅, -OH, fluor, chlore, -NO₂, -CN, -CHO, -CO-(alkyle en C₁₋₄), -CO-phényle, -COO-(alkyle en C₁₋₄), -NHCO-(alkyle en C₁₋₄), -CF₃, -NH₂, -NH-(alkyle en C₁₋₄) ou -N(alkyle en C₁₋₄)₂,
R¹ et R³ peuvent avoir, indépendamment l'un de l'autre, la même signification que R² et
R⁴ représente l'hydrogène, phényle, alkyle en C₁₋₈, -OH, fluor, chlore, -NO₂, -CN, -CHO ou -O-CO-(alkyle en C₁₋₄), par réaction des aromatiques chlorés de formule (II) : 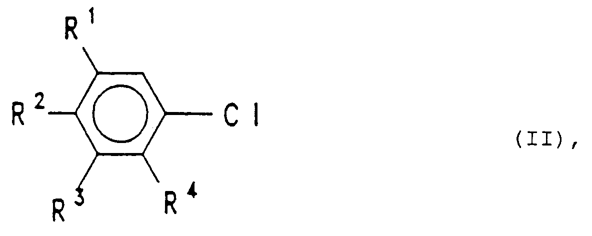
où R¹, R², R³ et R⁴ ont la signification précitée, avec des oléfines de formule (III) :
HX (III),
où X a la signification citée, en présence d'une base et de quantités catalytiques d'un complexe de palladium, caractérisé en ce que le complexe de palladium contient des phosphanes mono- et bidentés comme ligands.

2. Procédé selon la revendication 1, caractérisé en ce que l'on utilise une oléfine de formule (III) où X représente -C(R⁵)=C(R⁶)-Y et au moins l'un des radicaux R⁵ et R⁶ est l'hydrogène.

3. Procédé selon la revendication 1 ou 2, caractérisé en ce que l'on utilise un composé de formule (II), où R⁴ représente l'hydrogène et R¹ à R³, indépendamment l'un de l'autre, représentent l'hydrogène, phényle, méthyle, méthoxy, acétoxy, -NO₂, -CN, -CHO, -Cl, -NHCO-(alkyle en C₁₋₂), -COO-(alkyle en C₁₋₂), -CO-(alkyle en C₁₋₂), -CO-phényle ou -N(alkyle en C₁₋₂)₂.

4. Procédé selon la revendication 1 ou 2, caractérisé en ce que l'on utilise un composé de formule (II), où R¹, R³ et R⁴ représentent l'hydrogène ou méthyle et R² représente l'hydrogène, phényle, méthyle, méthoxy, acétoxy, -CN, -NO₂, -COOC-(alkyle en C₁₋₂), -CHO, -Cl ou -COCH₃.

5. Procédé selon une ou plusieurs des revendications 1 à 4, caractérisé en ce que l'on utilise en tant que composés de formule (III) styrène, éthylène, propylène, acrylonitrile, acrylate de méthyle, d'éthyle, de butyle et de 2-éthylhexyle, N,N-diméthyl- ou N,N-diéthylacrylamide.

6. Procédé selon une ou plusieurs des revendications 1 à 5, caractérisé en ce que l'on prépare 4-méthoxy-, 4-acétyl-, 4-formyl-, 4-nitro- ou 4-cyanocinnamate de butyle et d'éthylhexyle, 4,4'-dinitro-, 4-cyano- ou 4,4'-dicyanostilbène ou 4-cyanostyrène.

7. Procédé selon une ou plusieurs des revendications 1 à 6, caractérisée en ce que l'on met en oeuvre la réaction à une température comprise entre 120 et 200 °C, de préférence entre 140 et 180 °C.

8. Procédé selon une ou plusieurs des revendications 1 à 7, caractérisé en ce que le complexe de palladium se forme à partir de sels de palladium(II) et/ou des complexes de palladium(0) et de phosphanes mono- et bidentés dans le mélange réactionnel.

9. Procédé selon une ou plusieurs des revendications 1 à 7, caractérisé en ce que l'on prépare le complexe de palladium à partir des sels de palladium(II) et/ou de complexes de palladium(0) et de phosphanes mono- et bidentés séparément, et on réduit *in situ* le complexe de palladium(II)-phosphane obtenu en complexe de palladium(0)-phosphane correspondant.

10. Procédé selon une ou plusieurs des revendications 1 à 9, caractérisé en ce que le rapport molaire des atomes de phosphore aux atomes de palladium dans le catalyseur est compris entre 1,5:1 et 8:1, de préférence entre 1,6:1 et 6:1, plus particulièrement entre 2:1 et 5:1.

11. Procédé selon une ou plusieurs des revendications 1 à 10, caractérisé en ce que l'on utilise un catalyseur dans lequel le rapport molaire des phosphanes monodentés aux atomes de palladium est compris entre 0,5:1 et 4:1 et de biphosphanes bidentés aux atomes de palladium est compris entre 0,5:2 et 2:1.

12. Procédé selon une ou plusieurs des revendications 1 à 11, caractérisé en ce que le rapport molaire des phosphanes bidentés aux phosphanes monodentés est compris entre 1:1 et 1:4, de préférence entre 1:1,5 et 1:3, plus particulièrement entre 1:2.

13. Procédé selon une ou plusieurs des revendications 1 à 12, caractérisé en ce que l'on utilise un complexe de palladium qui contient en tant que ligands phosphane bidenté bis-(diphénylphosphino)-éthane, bis-(diphénylphosphino)-propane ou bis-(diphénylphosphino)-butane.

14. Procédé selon une ou plusieurs des revendications 1 à 13, caractérisé en ce que l'on utilise un complexe de palladium qui contient en tant que ligands phosphane monodentés triphénylphosphane, tricyclohexyl-phosphane ou le tri-o-tolylphosphane.

15. Procédé selon une ou plusieurs des revendications 1 à 14, caractérisé en ce que le complexe de palladium est calculé en tant que complexe de palladium mononucléé et utilisé en une quantité de 0,001 à 10 % en moles, de préférence de 0,1 à 1 % en moles par rapport aux aromatiques chlorés(II).

16. Procédé selon une ou plusieurs des revendications 1 à 15, caractérisé en ce que l'on utilise en tant que base l'acétate de sodium.
